(19) Europäisches Patentamt
European Patent Office
Office européen des brevets

(11) EP 4 536 675 B1

(12) EUROPEAN PATENT SPECIFICATION

(45) Date of publication and mention of the grant of the patent:
**21.01.2026 Bulletin 2026/04**

(21) Application number: **23738863.2**

(22) Date of filing: **12.06.2023**

(51) International Patent Classification (IPC):
***C07J 1/00*** *(2006.01)*

(52) Cooperative Patent Classification (CPC):
**C07J 1/007; C07J 1/0074**

(86) International application number:
**PCT/IB2023/056042**

(87) International publication number:
**WO 2023/242712 (21.12.2023 Gazette 2023/51)**

# (54) PROCESS FOR THE PURIFICATION OF 2-METHOXYESTRADIOL FROM 4-METHOXYESTRADIOL AND INTERMEDIATES OF THE PROCESS

VERFAHREN ZUR REINIGUNG VON 2-METHOXYESTRADIOL AUS 4-METHOXYESTRADIOL UND ZWISCHENPRODUKTE DES VERFAHRENS

PROCÉDÉ DE PURIFICATION DE 2-MÉTHOXYESTRADIOL À PARTIR DE 4-MÉTHOXYESTRADIOL ET INTERMÉDIAIRES DU PROCÉDÉ

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **13.06.2022 IT 202200012425**
**10.10.2022 IT 202200020784**

(43) Date of publication of application:
**16.04.2025 Bulletin 2025/16**

(73) Proprietor: **Industriale Chimica S.r.l.**
**20145 Milano (IT)**

(72) Inventors:
- **LENNA, Roberto**
  **20010 S. GIORGIO SU LEGNANO (IT)**
- **DELFRATE, Claudio**
  **20017 RHO (IT)**
- **RIGAMONTI, Davide**
  **22040 LURAGO D'ERBA (IT)**
- **BOGHI, Michele**
  **21056 Induno Olona (IT)**

(74) Representative: **Palladino, Saverio Massimo et al**
**Notarbartolo & Gervasi S.p.A.**
**Viale Achille Papa, 30**
**20149 Milano (IT)**

(56) References cited:
**WO-A2-01/14405 US-B1- 7 037 907**

- **CHEN S-H ET AL: "A new synthetic route to 2- and 4-methoxyestradiols by nucleophilic substitution", STEROIDS, ELSEVIER SCIENCE PUBLISHERS, NEW YORK, NY, US, vol. 47, no. 1, 1 January 1986 (1986-01-01), pages 63 - 81, XP023583051, ISSN: 0039-128X, [retrieved on 19860101], DOI: 10.1016/0039-128X(86)90077-2**
- **FISHMAN J: "Synthesis of 2-Methoxyestrogens", JOURNAL OF THE AMERICAN CHEMICAL SOCIETY, AMERICAN CHEMICAL SOCIETY, vol. 80, 1 January 1958 (1958-01-01), pages 1213 - 1216, XP002200986, ISSN: 0002-7863, DOI: 10.1021/JA01538A050**
- **KIURU P S ET AL: "Short synthesis of 2-methoxyestradiol and 2-hydroxyestradiol", STEROIDS, ELSEVIER SCIENCE PUBLISHERS, NEW YORK, NY, US, vol. 68, no. 4, 1 April 2003 (2003-04-01), pages 373 - 375, XP004429316, ISSN: 0039-128X, DOI: 10.1016/S0039-128X(03)00035-7**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### FIELD OF THE INVENTION

The present invention refers to the sector of the processes for the preparation of active ingredients for pharmaceutical use, and in particular, in the first aspect thereof, to an industrially applicable process for the purification of (17β)-2-methoxy-estra-1,3,5(10)-triene-3,17-diol, compound of interest for its known antiproliferative characteristics, from (17β)-4-methoxy-estra-1,3,5(10)-triene-3,17-diol; the two compounds are also known respectively under the names 2-methoxyestradiol and 4-methoxyestradiol, names with which they are generally referred to in the sector and which will be adopted in the following description.

In a second aspect thereof, the invention refers to intermediates of such a process.

### BACKGROUND

2-Methoxyestradiol has been described in the article "Demonstration of 2-methoxyestrone and of 2-methoxyestradiol in human pregnancy urine", by V. A. Frandsen, Acta Endocrinologica (1959), vol. 31, pages 603-7.

The structure of 2-methoxyestradiol is reported below:

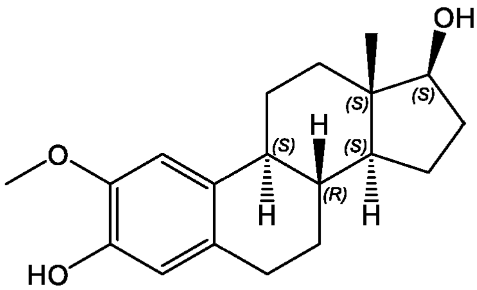

.

In the literature the compound is identified with CAS number 362-07-2.

2-Methoxyestradiol is a molecule of interest in particular since it prevents the formation of new blood vessels that tumours need to grow (angiogenesis), and induces apoptosis in some tumour cell lines, as reported in the article "The impact of endogenous estradiol metabolites on carcinogenesis", T. H. Lippert et al., Steroids (2000), vol. 65, pages 357-69.

Numerous processes of synthesis of 2-methoxyestradiol are described in the literature; one of the most recent is reported in the article "Short synthesis of 2-methoxyestradiol and 2-hydroxyestradiol", P. S. Kiuru et al., Steroids (2003), vol. 68, pages 373-375.

In this synthesis an isomer of 2-methoxyestradiol, 4-methoxyestradiol, is produced, which has CAS number 26788-23-8 and the formula reported below:

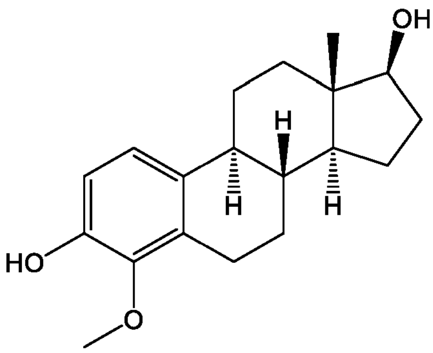

.

4-Methoxyestradiol is seriously suspected of generating a carcinogenic and mutagenic metabolite in vivo, as reported in the articles "4-Methoxyestradiol-induced oxidative injuries in human lung epithelial cells", Y. H. Cheng et al., Toxicology and Applied Pharmacology (2007) vol. 220, pages 271-277, and "Estradiol 17β and its metabolites stimulate cell proliferation and antagonize ascorbic acid-suppressed cell proliferation in human ovarian cancer cells", H.-H. Li, et al., Reproductive Sciences (2014) vol. 21, pages 102-111.

The formation of high amounts of 4-methoxyestradiol in the process described in the mentioned article of Steroids dated 2003 is in accordance with what is reported in the experimental part of the article itself: although no

indication is provided relating to the analytical profile of either the raw 2-methoxyestradiol or the 2-methoxyestradiol obtainable after chromatographic purification and crystallization, the purification yield of 64% (example 2.1.3 on page 374) is indicative of a low level of initial purity.

The synthesis process described in this article has also been reproduced several times by the present inventors, applying in the various preparations minimal variations to the recipes in order to define the optimal reaction parameters, but has led in all cases to mixtures with a content of 4-methoxyestradiol well above 5%, and in some cases even above 10%, with respect to the content of 2-methoxyestradiol, while the international guidelines in the pharmaceutical field have as maximum threshold a content of less than or equal to 0.15% for the identified impurities, and less than or equal to 0.10% for not identified ones.

Processes alternative to that of the article of Steroids dated 2003 are known, which lead to lower or even very low contents of 4-methoxyestradiol, but they are complicated and low-yielding processes, and still give rise to significant amounts of other impurities.

Patent application WO 01/14405 A2 describes in an extremely detailed manner the chromatographic purification of 2-methoxyestradiol from a series of impurities including 4-methoxyestradiol, using silica gel and mixtures of organic solvents that are predominantly chloroform-based as eluent; see preferred embodiment on page 16 of the text. In particular, 2-methoxyestradiol is purified from 4-methoxyestradiol (Table 1 and Example 2 on page 18) but starting from an already good level of purity, using silica gel in an amount equal to 171 times the weight of the mixture to be purified, and with the use of 2.5 L of eluent mixture, which is equivalent in the example to a ratio between the volume of eluent and the weight of the sample equal to 0.714 L/g; reporting these values on an industrial scale, 8.5 tons of silica gel eluted with 35700 L of solvent mixture would be required for the purification of a 50 kg batch of 2-methoxyestradiol; if in addition to this, account is taken of the elution times, the evaporation times of the solvent to recover the product, the analytical support of control of the fractions, and the disposal of the spent silica gel and solvent, it is immediately clear that this purification methodology is of no use in view of an application to industrial production batches, due to the laboriousness of the chromatographic technique, the quantities of materials to be used and the execution times.

Steroids Vol 47(1) pp 63-81 (1986) discloses a process whereby 2-methoxy- and 4-methoxy -estradiol are prepared by pre-emptively separating the 2-bromo- and 4-bromo- precursors thereof, rather than by the direct separation of the 2-methoxy and 4-methoxy compounds themselves.

The inventors studied possible alternative purification methods, chromatographic (but being less onerous than that of WO 01/14405 A2), by crystallizations, or combinations of these techniques. The experimentation was carried out on samples of 2-methoxyestradiol with a 4-methoxyestradiol content higher than 5%, which were obtained with the process of the mentioned article of Steroids dated 2003. With regard to the chromatographic methods, various eluent mixtures composed of alcohols, chlorinated solvents, ketones and ethers have been tried, such as various dichloromethane/-methanol mixtures with a weight ratio between silica gel and product between 50:1 and 200: 1, a dichloromethane/acetone mixture 98:2 with a weight ratio between silica gel and product equal to 100:1, a chloroform/methanol mixture 99:1 with a weight ratio between silica gel and product equal to 200:1, and diisopropyl ether-ethyl ether mixtures with a gradient from 70:30 to 0:100 and a weight ratio between silica gel and product equal to 200:1.

As far as the crystallizations are concerned, these were tried using various pure solvents or in mixture with each other; specifically, crystallization was tried from the pure solvents methanol, acetonitrile, isopropyl acetate, ethyl acetate, acetone, methyltetrahydrofuran, methylethylketone (MEK), ethanol, isopropanol, toluene, methyl ter-butyl ether (MTBE), diisopropylether, chloroform, dichloromethane (DCM) and acetic acid, and from the mixtures DCM/methanol, MTBE/tetrahydrofuran (tetrahydrofuran is generally abbreviated as THF), MTBE/1,4-dioxane, MTBE/1-pentanol, MTBE/2-methoxy-tetrahydropyranyl (tetrahydropyranyl is generally abbreviated as THP), chloroform/1-pentanol, chloroform/2-methoxy-THP, chloroform/3-methyl-1-butanol, acetonitrile/THF, acetonitrile/THF/3-methyl-1-butanol, methanol/THF, methanol/THF/3-methyl-1-butanol, acetonitrile/THF/1,2-dimethoxyethane, acetonitrile/THF/2-ethoxyethanol, chloroform/THF, chloroform/methanol, dichloromethane/acetonitrile, dichloromethane/ethyl acetate and water/dimethylformamide/-methanol.

In all cases negative results were obtained: the best result was the lowering of the content of 4-methoxyestradiol to about 5% by crystallizing from MTBE; subsequent crystallizations from MTBE however did not lead to further improvements in the content of 4-methoxyestradiol.

It was therefore not possible, despite the extensive experimental testing campaign, to identify a technique of the chromatographic type (both flash and gravimetric), of crystallization, or a combination thereof, which would allow to reach the target of a content of 4-methoxyestradiol of less than 0.15%, as required by the international guidelines.

Object of the present invention is to make available a methodology of purification of the mixture composed of 2-methoxyestradiol and of 4-methoxyestradiol as main contaminant, which is of real applicability in a process on industrial scale.

## SUMMARY OF THE INVENTION

This aim is reached by the present invention, which concerns a process for the purification of 2-methoxyestradiol from 4-methoxyestradiol comprising the following steps:

a) reaction of a mixture (N-3) comprising 2-methoxyestradiol and 4-methoxyestradiol with:

a') a carboxylic acid selected from formic acid, acetic acid and propionic acid; or
a") the derivative of a carboxylic acid selected from an ester, an anhydride, an acyl chloride and an acyl bromide, obtaining:

- in case a') a mixture N-2' comprising the corresponding monoesters in position 17 of the steroid skeleton:

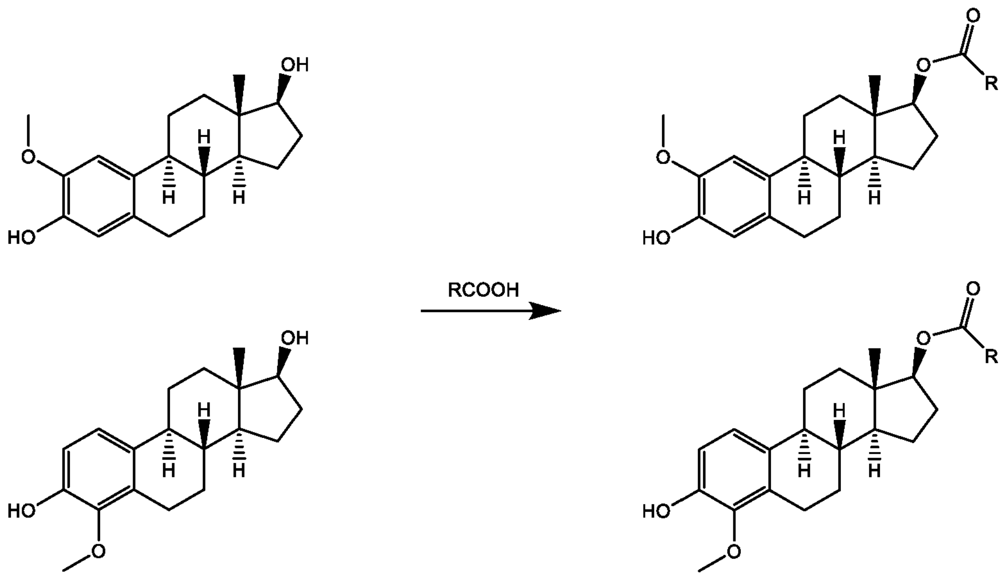


**N-3** **N-2'** ;

- in case a") a mixture N-2" comprising the corresponding diesters in positions 3 and 17 of the steroid skeleton:

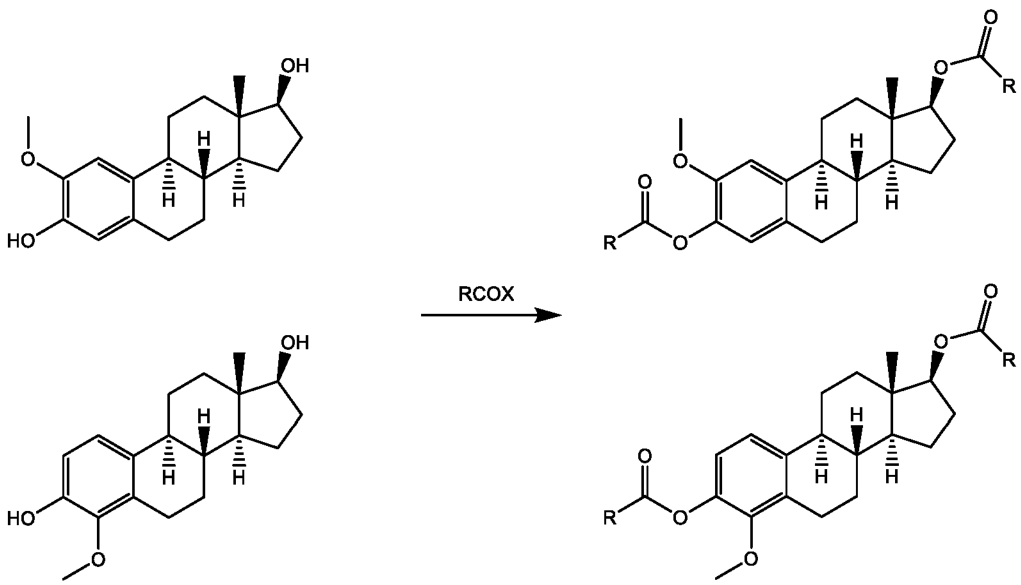


**N-3** **N-2"**

wherein X is O-alkyl or O-aryl in case of esters, OCO-alkyl in case of anhydrides, and Cl or Br in case of acyl chlorides and bromides;

b) separation by crystallization of derivative N-1' or of derivative N-1" from mixture N-2' or N-2", respectively:

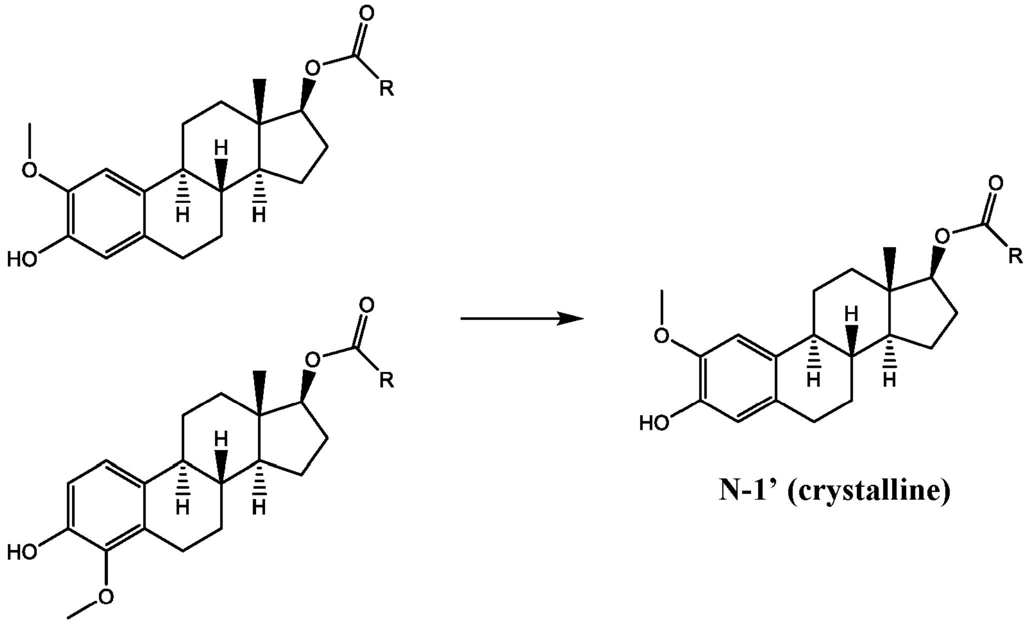

N-1' (crystalline)

N-2'

;

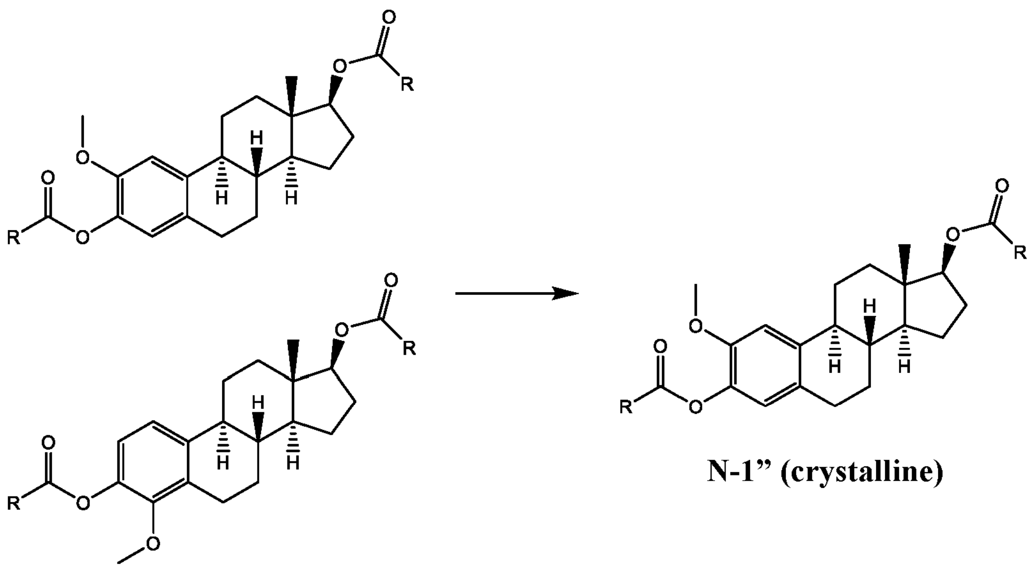

N-1" (crystalline)

N-2"

;

c) hydrolysis of derivative N-1' or of derivative N-1" to yield 2-methoxyestradiol:

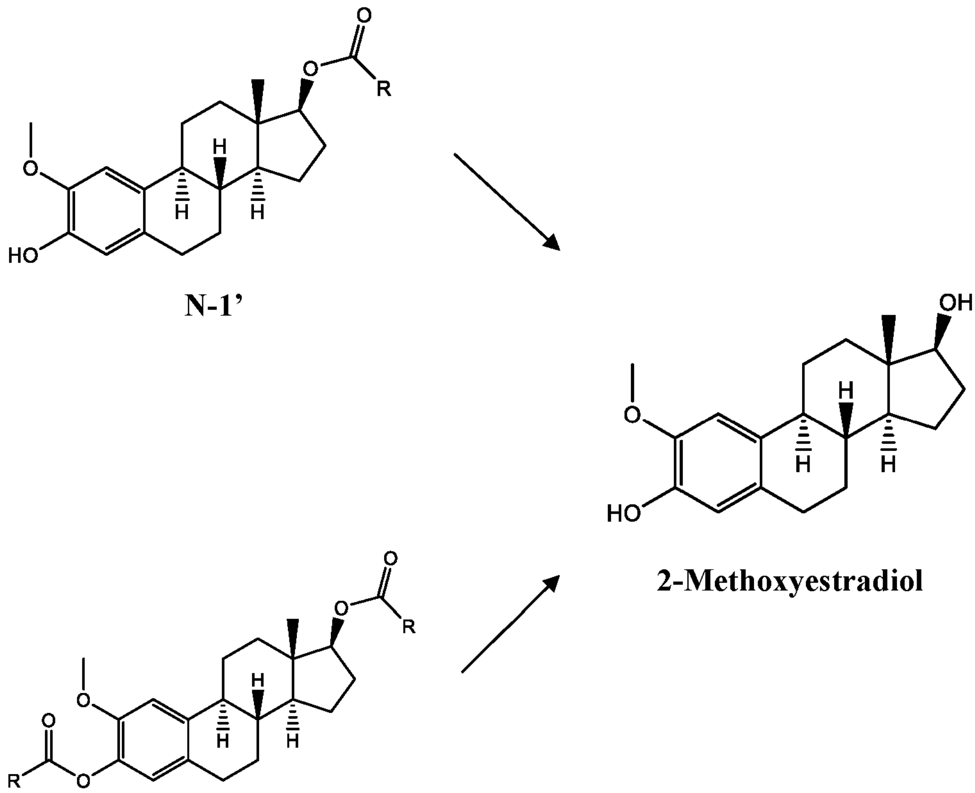

N-1'

2-Methoxyestradiol

N-1"

.

In its second aspect, the invention concerns the compounds 2-methoxy-3-hydroxyestra-1,3,5(10)-triene-17β-yl formate, having the following formula:

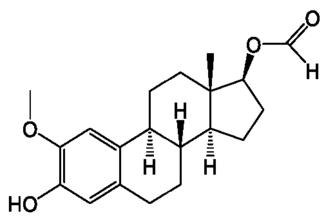

,

and 2-methoxy-3-hydroxyestra-1,3,5(10)-triene-3,17β-yl difuroate, having the following formula:

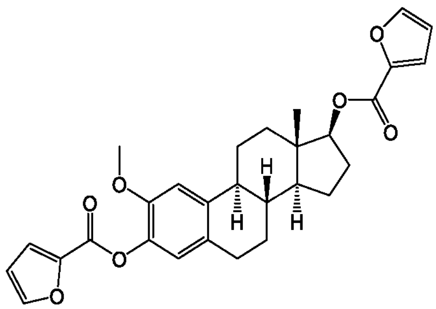

.

**DETAILED DESCRIPTION OF THE INVENTION**

During the process of the invention, some mixtures of reagents or products in solvents are in the form of suspensions or solutions in the various steps of the process itself; the term "mixture" is then used below to indicate both a suspension and a solution.

In the first aspect thereof, the invention concerns a process for the purification of 2-methoxyestradiol from 4-methoxyestradiol, which allows to reduce the content of 4-methoxyestradiol to values lower than 0.15% with respect to the content of 2-methoxyestradiol (area % HPLC).

The process of the invention comprises the steps a) to c) reported above.

In step a) a mixture, N-3, is formed, comprising 2-methoxyestradiol and 4-methoxyestradiol and a compound capable of esterifying one or both of the hydroxyl groups of the two diols.

This step can be carried out according to the two alternative modes a') and a") indicated above.

According to the mode a'), the esterification is carried out with an acid selected from formic acid, acetic acid and propionic acid; in this mode, the acid itself acts as a solvent.

Preferably formic acid or acetic acid are used.

Initially, the mixture N-3 consists of a suspension of 2-methoxyestradiol and 4-methoxyestradiol in the acid; the two compounds are then solubilized, forming a solution. In the solution thus formed the esterification reaction takes place; the inventors have observed that, surprisingly, by operating according to this mode the esterification is completely regioselective, and as a result of the reaction only the esters in position 17 of the steroid skeleton are obtained.

The acid used for the formation of the mixture N-3 may be pure or diluted with water; preferably an acid with a degree of purity higher than or equal to 95%, more preferably higher than or equal to 98% is used.

The ratio between mL of acid and grams of the N-3 mixture is between 4 and 8, preferably between 5.5 and 6.5.

This step is carried out at a temperature between 10 and 90 °C, preferably between 20 and 80 °C.

To facilitate the esterification reaction it is possible, but not necessary in mode a'), to use para-toluenesulfonic acid as a catalyst.

The inventors have observed that upon formation of the N-3 mixture, realized by adopting the weight ratios between acid and 2-methoxyestradiol/4-methoxyestradiol and in the temperature ranges indicated above, in this step the spontaneous crystallization and selective precipitation of the 17-ester of 2-methoxyestradiol takes place, which forms a new suspension, while the 17-ester of 4-methoxyestradiol remains in solution. The esterification reaction of 2-methoxyestradiol and of 4-methoxyestradiol and the selective precipitation of the 17-ester of 2-methoxyestradiol proceed simultaneously during step a').

The precipitation by crystallization of the 17-ester of 2-methoxyestradiol, compound N-1', takes place from the mixture N-2' without the need for addition of any solvent with the function of lowering the solubility thereof. It is also possible to induce precipitation of the 17-ester of 2-methoxyestradiol by adding a small amount of the same compound (crystallization germ or seed) obtained in a previous preparation; however, the spontaneous crystallization of the 17-ester of 2-methoxyestradiol, N-1', from the reaction mixture is preferably awaited.

By operating according to mode a"), the esterification is carried out with a derivative of an acid selected from an ester, an anhydride, an acyl chloride or bromide.

The general composition of the reagent mixture of step a") comprises 2-methoxyestradiol (containing variable percentages of 4-methoxyestradiol, mixture N-3), organic solvent, an organic base, 4-dimethylaminopyridine (4-DMAP) as a catalyst and the derivative of the carboxylic acid.

The acyl bromides and even more preferably the acyl chlorides are preferably used as derivatives of the carboxylic acids.

The acyl chloride is selected from furoyl chloride and benzoyl chloride, with a degree of purity higher than or equal to 95%, more preferably higher than or equal to 98%.

The organic solvent, inert to the reaction conditions, is selected from: tetrahydrofuran, dichloromethane, chloroform, hexane, heptane, dimethylformamide, dimethylacetamide. Preferably dichloromethane and tetrahydrofuran are used.

The organic base is selected from triethylamine, trimethylamine, pyridine; preferably triethylamine is used.

Step a") is carried out at a temperature between 10 and 50 °C, preferably between 20 and 30 °C.

The ratio between the volume of the solvent in mL and the weight of the N-3 mixture in g is between 10 and 25, preferably between 15 and 20.

The ratio between the volume of the solvent and that of the base is between 3 and 6.5, preferably between 4 and 5.5.

The volume of solvent is at least 10 times the volume of the used acyl chloride.

The ratio between the weight of the N-3 mixture and the weight of the catalyst (4-DMAP) is between 20 and 30, preferably between 23 and 27.

The reagent mixture of step a") is heated and refluxed for a time interval between 12 and 36 hours, preferably between 16 and 30 hours during which, unlike of what happens in the regioselective reaction of step a'), the esterification reaction takes place on both the hydroxyls in position 3 and 17 of the steroid skeleton.

Step b) is carried out according to different modes depending on whether it is started from the mixture N-2' or N-2".

In the first case, in step b), the 17-ester of 2-methoxyestradiol (derivative N-1') is separated from the mixture N-2'. The recovery of the derivative N-1' can be carried out by known methods, for example by centrifugation or preferably by simple filtration.

In the second case, in step b) the separation of the 3,17-diester N-1" from the mixture N-2" takes place by crystallization from methyl ter-butyl ether (MTBE) after work-up of the reaction mixture itself.

It is also possible to induce the precipitation of the 3,17-diester of 2-methoxyestradiol by adding a small amount of the same compound (crystallization germ or seed) obtained in a previous preparation; however, the spontaneous crystallization of the 3,17-diester of 2-methoxyestradiol, N-1", from MTBE is preferably awaited.

Finally, in step c) the intermediate N-1' or N-1" is hydrolyzed to yield 2-methoxyestradiol.

Even in case of step c) the operating modes vary slightly in the case where the hydrolysis is carried out on the intermediate N-1' or on the intermediate N-1".

The hydrolysis of the 17-ester of 2-methoxyestradiol (N-1') is carried out in an alcoholic solvent selected from methanol, ethanol and isopropanol, pure or in mixture with each other, anhydrous or in the presence of water. Preferably pure methanol with a content of water of less than 5% by volume is used, and more preferably pure and anhydrous methanol is used.

The base used for the hydrolysis reaction is selected from lithium hydroxide, sodium hydroxide, potassium hydroxide, lithium carbonate, sodium carbonate, potassium carbonate and cesium carbonate; preferably sodium hydroxide is used.

The reaction temperature is between 15 and 45 °C, preferably between 20 and 40 °C.

The amount of base used is such that the pH of the solution is between 8 and 14, preferably between 11 and 13.

The hydrolysis of the 3,17-diester of 2-methoxyestradiol (N-1") is carried out in a solvent selected from tertrahydrofuran, methanol, ethanol and isopropanol, pure or in mixture with each other, anhydrous or in the presence of water. Preferably, tetrahydrofuran and methanol pure or in mixture with each other are used.

The base used for the hydrolysis reaction is selected from lithium hydroxide, sodium hydroxide, potassium hydroxide, lithium carbonate, sodium carbonate, potassium carbonate and cesium carbonate; preferably sodium hydroxide is used.

The reaction temperature is between 20 °C and the reflux temperature of the reaction mixture.

The amount of base used is such that the pH of the solution is between 8 and 14, preferably between 11 and 13.

In the second aspect thereof, the invention concerns the following intermediate compounds of the process:

- 2-methoxy-3-hydroxyestra-1,3,5(10)-triene-17β-yl formate, having the following formula:

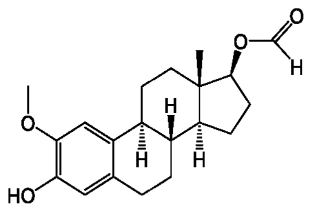

;

- 2-methoxy-3-hydroxyestra-1,3,5(10)-triene-3,17β-yl difuroate, having the following formula:

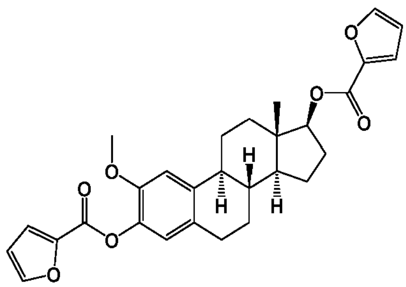

.

The invention will be further illustrated by the following examples.

## EXPERIMENTAL INSTRUMENTS, METHODS AND CONDITIONS

### *NMR*

NMR spectrometer JEOL 400 YH (400 MHz); JEOL Delta software v5.1.1;
Spectra recorded in deuterated solvents such as: Chloroform-d, D 99.8%, containing 0.1% (v/v) tetramethylsilane (TMS) as internal standard; and Chloroform-d, "100%", D 99.96%, containing 0.03% (v/v) TMS, and DMSO-$d_6$.

***MS***

Spectrometer WATERS SYNAPT G2-Si QTof
Negative ionization
Ionization is done in ESI (electron spray ionization)

***HPLC***

Agilent HPLC model 1260 Infinity II
UV-DAD detector
*Mobile phase:* Water / Acetonitrile / Methanol 55:40:5 (v/v)

*Chromatographic conditions:*

| | | |
|---|---|---|
| Column | : | Supelco Ascetis Express C18, 150 x 4.6 mm, 2.7 μm |
| Flow | : | 1.0 mL/minute |
| Detector | : | UV at 204 and 280 nm |
| Injection volume | : | 10 μL |
| Temperature | : | 25 °C. |

***UPLC***

Chromatographic System: Waters Acquity UPLC;
Detector: Acquity UPLC PDA and λ Detector (λ = 220nm)
Column: Acquity UPLC BEH C18 1.7 μm (2.1×50 mm), T = 35 °C
Solvent A: Acetonitrile 0.01% formic acid
Solvent B: water 0.01% formic acid
Gradient:

| | Time (min) | Flow (mL/min) | %A | %B |
|---|---|---|---|---|
| 1 | 0.0 | 0.5 | 30 | 70 |
| 2 | 0.5 | 0.5 | 30 | 70 |
| 3 | 4.0 | 0.5 | 90 | 10 |
| 4 | 4.1 | 0.5 | 30 | 70 |
| 5 | 5.5 | 0.5 | 30 | 70 |

***Chromatographic purity (Ph Eur):***

The chromatograms obtained from the "HPLC Titre" test, carried out by liquid chromatography under the following conditions, defined in Ph Eur 2.2.29, are used:

*Solvent:* Acetonitrile / Water 70:30 v/v
*Reference solution A:* dissolve 20 mg of 2-methoxy-estradiol Working STD in 14 mL of acetonitrile. Dilute to 20 mL with water. Prepare this solution in duplicate.
*Sample solution:* dissolve 20 mg of product in 14 mL of acetonitrile, dilute to 20 mL with water. Prepare this solution in duplicate.

*Reference solution B:* take 1 mL of sample solution and dilute to 100 mL with solvent. From this solution dilute 1 mL to 10 mL with solvent.

Identify impurities based on their relative retention times:

| Name | RRT | RT |
|---|---|---|
| 17β-estradiol | 0.80 | 4.4 |
| 4-methoxy-estradiol | 0.87 | 4.8 |
| 2-methoxy-estradiol | 1 | 5.5 |

Calculate the content of the impurities, at 204 nm, according to the formula:

$$C_u\% = (r_u/r_s) \times 0.1$$

where:

$C_u\%$ is the content of each impurity
$r_u$ is the response area of each impurity
$r_s$ is the response area of 2-methoxy-estradiol in the reference solution B.

For the calculation of total impurities, discard all impurities lower than 0.05%.

***TLC***

MERCK: TLC silica gel 60 $F_{254}$ Aluminium sheets 20 x 20 cm, code 1.0554.0001.

***TLC detectors***

Cerium phosphomolybdate: 25 g of phosphomolybdic acid and 10 g of cerium (IV) sulphate are dissolved in 600 mL of $H_2O$. 60 mL of $H_2SO_4$ 98% are added and it is brought to 1 L with $H_2O$. The strip is impregnated with solution and then heated until the products are detected.

***REMARKS***

The water used in the experimental descriptions is to be understood as pure water unless otherwise indicated.
The organic solvents used in the experimental descriptions are to be understood as of "technical" grade unless otherwise indicated.
The reagents used in the experimental descriptions are to be understood as of commercial quality unless otherwise indicated.

**EXAMPLE 1**

This example refers to the process of the invention carried out by using a carboxylic acid in the esterification step.

Step a')

2-methoxyestradiol (33.8 g, previously crystallized from MTBE) with 4.7% content of 4-methoxyestradiol was suspended in a reactor with 202.8 mL of formic acid, under nitrogen, at 25 °C ± 5 °C.
The suspension thus formed was placed under stirring. After 35' the complete dissolution of the initial solid was observed. After 1 hr and 10' the formation of new solid in suspension was observed. After 2 hours the reaction status was checked by UPLC: 2-methoxyestradiol < 1%.

Step b)

The obtained suspension was filtered, the obtained solid (formate of 2-methoxyestradiol) was washed on a

buchner with formic acid (34 mL) and dried summarily always on a buchner (wet solid).

A sample of solid thus obtained, after drying under reduced pressure, was analysed by $^1$H-NMR and mass.

$^1$H-NMR (400MHz, DMSO-d6): 8.62 (1H, s, Ar-OH); 8.26 (1H, s, CHO); 6.76 (1H, s, Ar-H), 6.44 (1H, s, Ar-H); 4.68 (1H, t, H-17, J=8,5 Hz); 3.71 (3H, s, $OCH_3$); 2.73-2.57 (2H, m); 2.34-2.23 (1H, m); 2.20-2.08 (2H, m); 1.83-1.74 (2H, m); 1.73-1.63 (1H, m); 1.59-1.48 (1H, m); 1.44-1.14 (6H, m); 0.80 (3H, s, H-18).
Mass (ESI): m/z = $M^+$-1 = 329.

Step c)

The obtained wet solid was charged into a reactor with 6.7 g of sodium hydroxide in pellets.

Methanol (369 mL) was added and under stirring the complete dissolution of the initial solid was observed after 15'. The internal temperature rose from 25 to 32 °C, the measured pH was ≥ 12.

After further 30' of stirring the complete dissolution of the solid was observed and the state of the reaction was monitored by UPLC: complete conversion, formate of 2-methoxyestradiol < 1%.

An aqueous ammonium chloride solution (26 g in 1300 mL $H_2O$) was added to the reaction mixture.

The resulting hydroalcoholic mixture was then stirred at 25 °C ± 5 °C for 30' and then at 0-5 °C for 1 hr.

The formation of a precipitate was observed which was filtered on a buchner and washed with water on the filter.

The obtained solid was then oven-dried at 55 °C for 16 hrs under reduced pressure.

23.9 g of 2-methoxyestradiol (white solid with yellow reflections) with a content of 4-methoxyestradiol of less than 0.05% were obtained.

**EXAMPLE 2**

This example refers to the process of the invention carried out by using a second carboxylic acid in the esterification step.

Step a')

2-Methoxyestradiol (8 g) with 3.92% amount of 4-methoxyestradiol (HPLC) was suspended in 48 mL of glacial acetic acid with 453 mg of PTSA in a flask under nitrogen at 25 °C ± 5 °C.

The suspension was stirred and heated to 70 °C for 16 hours: after 2 hrs the solid was completely dissolved. After 16 hours the reaction was monitored by UPLC: 2-methoxyestradiol < 1%
The reaction mixture was cooled to 25 °C; reprecipitation was observed, and the mixture was kept under stirring for 1 hr.

Step b)

The solid was filtered and washed with acetic acid (8 ml) and dried under vacuum at 45 °C obtaining 5.85 g of powder.

Step c)

5.35 g of the solid obtained in step b) and 931 mg of sodium hydroxide were charged into a round-bottomed flask.

Methanol (53 ml) was added and the mixture was stirred for 3 hours at 20 < T (°C) < 25. The hydrolysis was monitored by UPLC: complete conversion.

An aqueous ammonium chloride solution (2.6 g in 54 ml of $H_2O$) was prepared.

The aqueous solution was added to the reaction mixture and the resulting hydroalcoholic mixture was stirred at 25 °C ± 5 °C for 30 minutes and then at 0-5 °C for 1 hour.

The precipitate was filtered and washed with water (65 ml).

The solid was oven-dried at 50 °C for 16 hrs obtaining 4.6 g of 2-methoxyestradiol in which the presence of 4-methoxyestradiol was not detectable.

**EXAMPLE 3**

This example refers to the process of the invention carried out by using a derivative of a carboxylic acid in the esterification step.

Step a")

In a flask under nitrogen, kept at a temperature of 25 °C ± 5 °C, 8 g of 2-methoxyestradiol containing 3.92% (HPLC) of 4-methoxyestradiol, 29.5 ml of triethylamine and 0.32 g of 4-dimethylaminopyridine (DMAP) were suspended in 160 ml of dichloromethane in a flask under nitrogen. Complete dissolution of the solids was not observed.
The mixture was cooled to 0 °C and 13.5 ml of benzoyl chloride were added dropwise. The temperature rose up to 13 °C and the formation of a precipitate was observed.
The mixture was then heated, refluxed and stirred for 16 hours. The reaction was then monitored by UPLC: 2-methoxyestradiol < 1%.

Step b)

The mixture obtained in step a") was cooled to 20 °C and poured onto 120 ml of ice-cold 1 M hydrochloric acid, keeping T < 20 °C. The mixture was then stirred for 20 minutes. The layers were separated and the aqueous phase was extracted with dichloromethane (30 ml). The organic phases were washed with an aqueous solution of $NaHCO_3$ and then with water (pH ≥ 7).
The organic layer was evaporated in vacuo obtaining an oil. MTBE (240 ml) was added and the system was subjected to evaporation until obtaining 60 ml of a suspension with a solid which was cooled and filtered, washing with MTBE having a temperature between 0 and 5 °C. The solid was dried at 50 °C under vacuum obtaining 9.7 g of white powder.

Step c)

300 mg of the solid obtained in step b) and 70 mg of sodium hydroxide were suspended with methanol (6 ml) and THF (1.5 ml) and heated and refluxed for 6 hrs (obtaining a clear solution); the reaction was then monitored by UPLC: 2-methoxyestradiol 3,17 dibenzoate < 1%.
The reaction mixture was cooled to room temperature and poured into an aqueous solution of ammonium chloride (5 g in 20 ml) and stirred for 20 minutes at room temperature and then for 1 hour at 0 °C, observing the formation of a solid which was filtered and washed with water. The solid was dried at 45 °C under vacuum obtaining 120 mg of 2-methoxyestradiol wherein 4-methoxyestradiol was not detectable on HPLC analysis.

**EXAMPLE 4**

This example refers to the process of the invention carried out by using a second derivative of a carboxylic acid in the esterification step.

Step a")

In a flask under nitrogen kept at 25 °C ± 5 °C, 8 g of 2-methoxyestradiol containing 3.92% (HPLC) of 4-methoxyestradiol, 29.5 ml of triethylamine and 0.32 g of 4-dimethylaminopyridine (DMAP) were dissolved in 120 ml of THF.
The mixture was cooled to 0 °C and 11.4 ml of furoyl chloride were added dropwise. The temperature rose up to 15 °C and the formation of a precipitate was observed.
The mixture was then heated, refluxed and stirred for 24 hours. The reaction was then monitored by UPLC: 2-methoxyestradiol < 1%.

Step b)

The mixture obtained in step a") was cooled to 20 °C and poured onto 120 ml of ice-cold 1 M hydrochloric acid, keeping T < 20 °C. Then 80 ml of isopropyl acetate were added and the mixture was stirred for 20 minutes. The layers were separated, the aqueous phase extracted with isopropyl acetate (2 x 50 ml). The organic phases were combined and washed with an aqueous solution of $NaHCO_3$ and then with water (pH ≥ 7).
The organic layer was evaporated in vacuo obtaining a yellow solid (18.6 g). The product was suspended in MTBE (54 ml), stirred at 20 °C for 20 minutes and then cooled to 0 °C. After 1 hour the solid was filtered and washed with MTBE having a temperature between 0 and 5 °C. The solid was dried at 50 °C under vacuum obtaining 10.27 g of white powder.

Step c)

200 mg of the solid obtained in step b) and 50 mg of sodium hydroxide were suspended with methanol (3 ml) and THF (1 ml) and stirred at 25 °C for 1.5 hrs obtaining a clear solution; the reaction was then monitored by UPLC: 2-methoxyestradiol 3.17 difuroyl < 1%.

The reaction mixture was poured into an aqueous solution of ammonium chloride (3.5 g in 10 ml) and stirred for 20 minutes at room temperature and then for 1 hour at 0 °C, observing the formation of a solid which was filtered and washed with water. The solid was dried at 50 °C under vacuum obtaining 90 mg of 2-methoxyestradiol wherein 4-methoxyestradiol was not detectable (HPLC).

## Claims

**1.** Process for the purification of 2-methoxyestradiol from 4-methoxyestradiol comprising the following steps:

a) reaction of a mixture (N-3) comprising 2-methoxyestradiol and 4-methoxyestradiol with:

a') a carboxylic acid selected from formic acid, acetic acid and propionic acid or
a") the derivative of a carboxylic acid selected from an ester, an anhydride, an acyl chloride and an acyl bromide,

obtaining:

- in case a') a mixture N-2' comprising the corresponding monoesters in position 17 of the steroid skeleton:

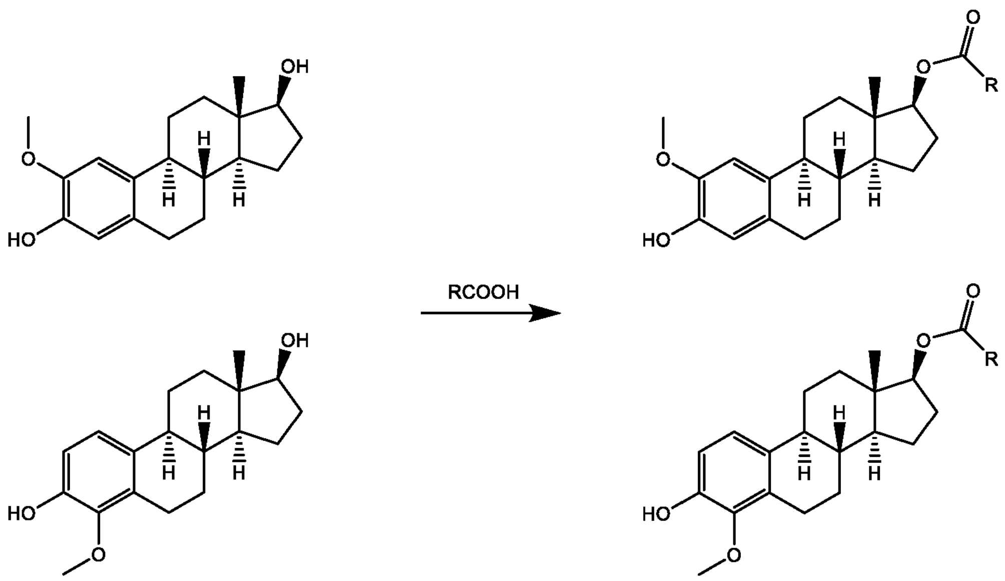


N-3 N-2' ;

- in case a") a mixture N-2" comprising the corresponding diesters in positions 3 and 17 of the steroid skeleton:

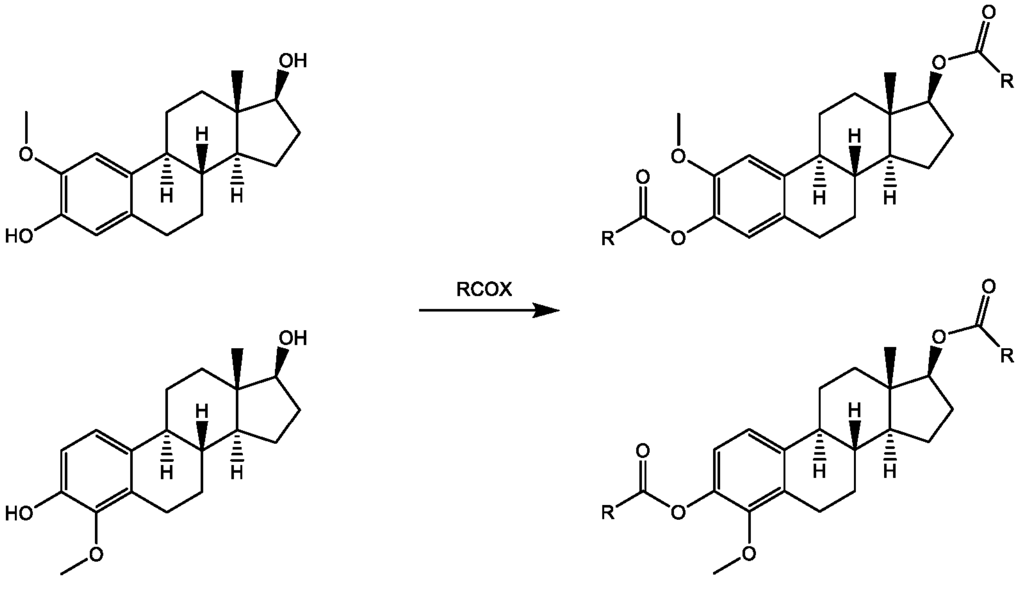


N-3

N-2”

wherein X is O-alkyl or O-aryl in case of esters, OCO-alkyl in case of anhydrides, and Cl or Br in case of acyl chlorides and acyl bromides;

b) separation by crystallization of derivative N-1’ or of derivative N-1" from mixture N-2’ or N-2", respectively:

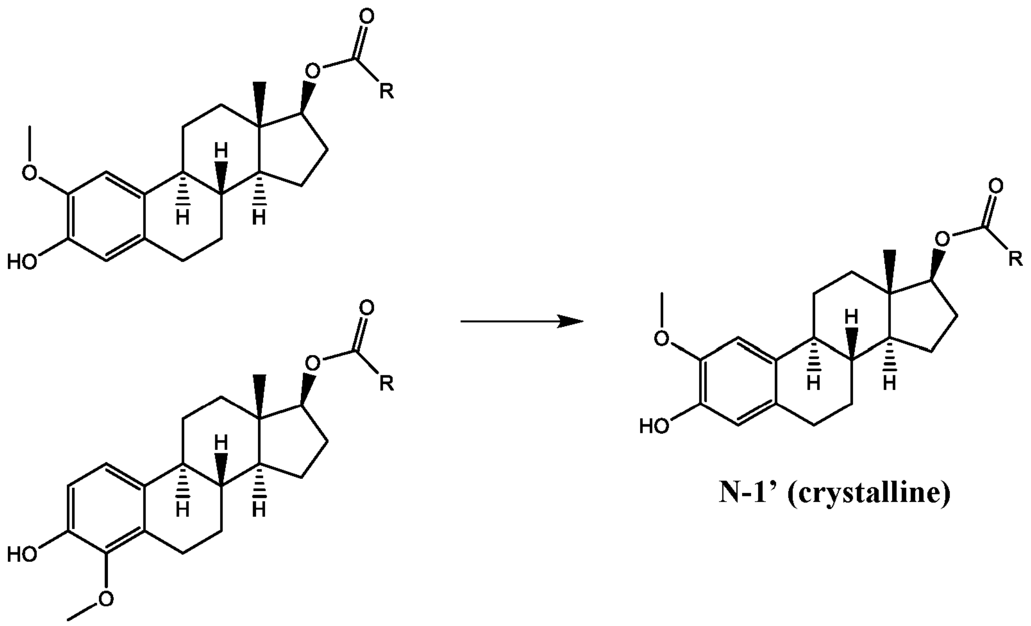

N-1’ (crystalline)

N-2’

;

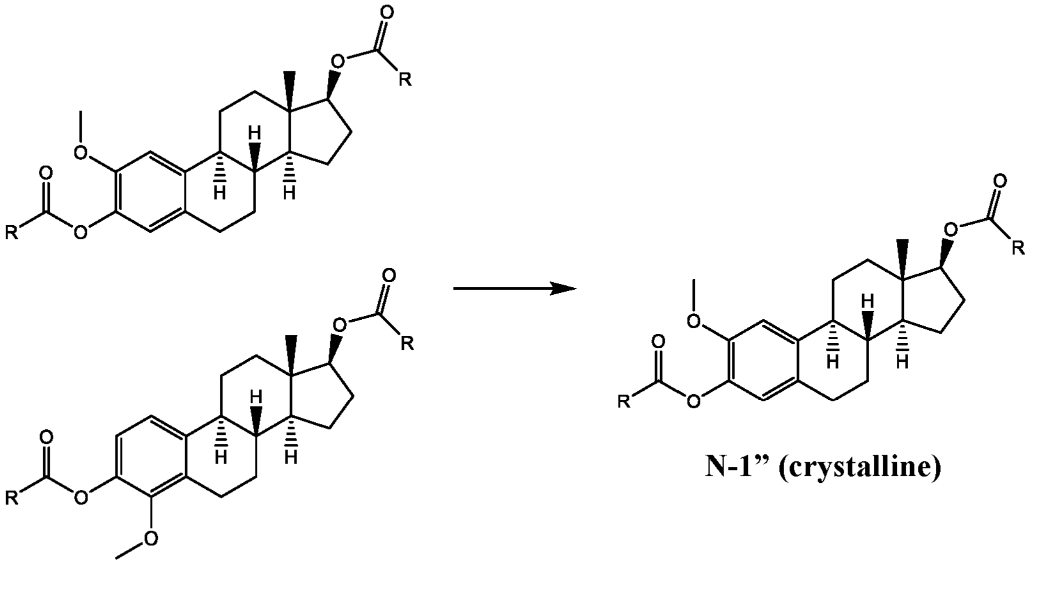

**N-1” (crystalline)**

**N-2”** ;

c) hydrolysis of derivative N-1’ or of derivative N-1" to yield 2-methoxyestradiol:

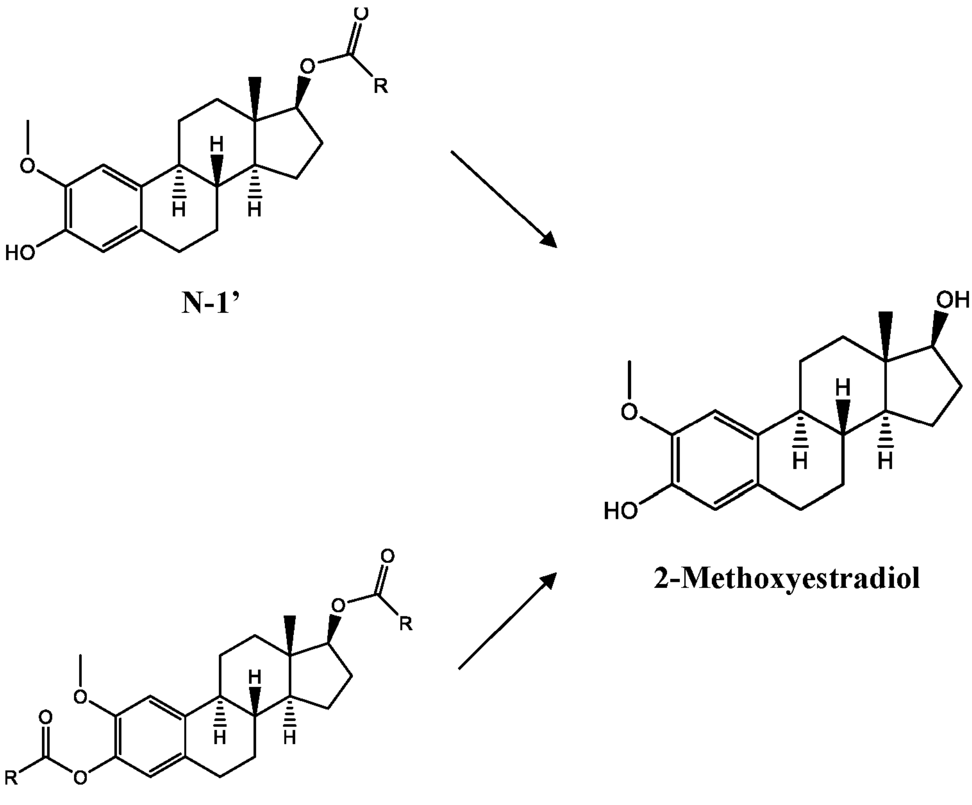

**N-1’**

**2-Methoxyestradiol**

**N-1”** .

**2.** Process according to claim 1, in which in step a’) the acid is selected between formic acid and acetic acid.

**3.** Process according to any one of claims 1 or 2, in which in step a’) the used acid acts as a solvent.

**4.** Process according to any one of the preceding claims, in which in step a’) the ratio between mL of acid and grams of the N-3 mixture is between 4 and 8 and said step a’) is carried out at a temperature between 10 and 90 °C.

5. Process according to any one of the preceding claims, in which in step a') para-toluenesulfonic acid is used as a catalyst.

6. Process according to claim 1, wherein step a") is carried out with a reagent mixture (N-3) comprising 2-methoxyestradiol and 4-methoxyestradiol, an organic solvent, an organic base, 4-dimethylaminopyridine and said derivative of a carboxylic acid.

7. Process according to claim 6 wherein said derivative of a carboxylic acid is selected from acyl chlorides and bromides.

8. Process according to claim 7 wherein said acyl chloride is selected from benzoyl chloride and furoyl chloride.

9. Process according to any one of claims 6 to 8, in which step a") is carried out at a temperature between 10 and 50 °C, the ratio between the volume of the solvent in ml and the weight of the N-3 mixture in g is between 10 and 25, the ratio between the volume of the solvent and that of the organic base is between 3 and 6.5, the volume of solvent is at least 10 times the volume of the acyl chloride used and the ratio between the weight of mixture N-3 and the weight of the 4-dimethylaminopyridine catalyst is between 20 and 30.

10. An intermediate of the process of claim 1, selected from:

- 2-methoxy-3-hydroxyestra-1,3,5(10)-triene-17β-yl formate, having the following formula:

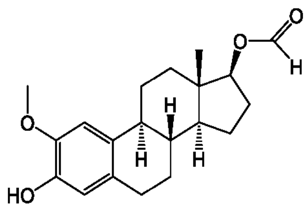
;

- 2-methoxy-3-hydroxyestra-1,3,5(10)-triene-3,17β-yl difuroate, having the following formula:

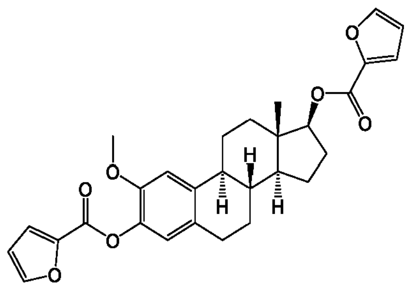
.

**Patentansprüche**

1. Verfahren zur Reinigung von 2-Methoxyestradiol aus 4-Methoxyestradiol, umfassend die folgenden Schritte:

a) Umsetzen eines Gemisches (N-3), das 2-Methoxyestradiol und 4-Methoxyestradiol umfasst, mit:

a') einer Carbonsäure, die aus Ameisensäure, Essigsäure und Propionsäure ausgewählt ist, oder
a") dem Derivat einer Carbonsäure, das aus einem Ester, einem Anhydrid, einem Acylchlorid und einem Acylbromid ausgewählt ist,
wobei erhalten wird:

- im Fall a') ein Gemisch N-2', das die entsprechenden Monoester an Position 17 des Steroidgerüsts umfasst:

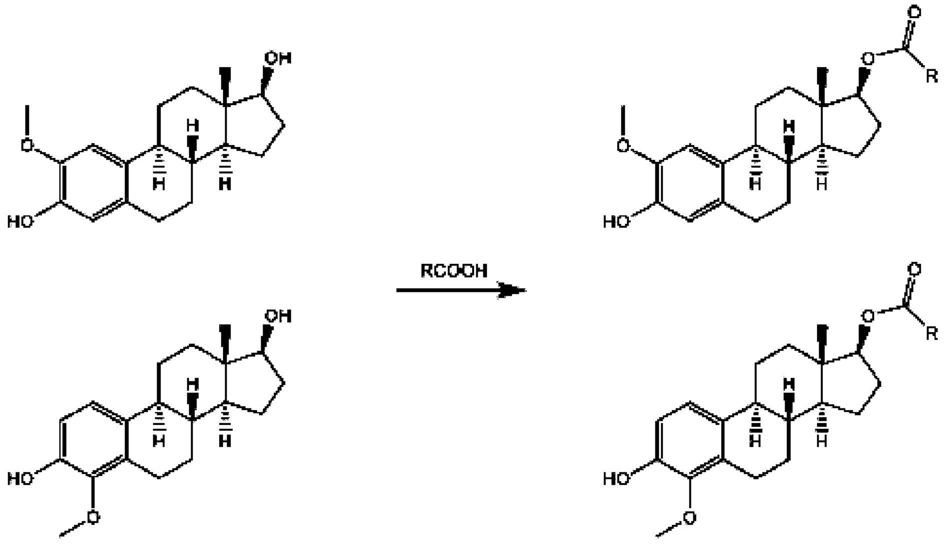


N-3 N-2' ;

- im Fall a") ein Gemisch N-2", das die entsprechenden Diester an den Positionen 3 und 17 des Steroidgerüsts umfasst:

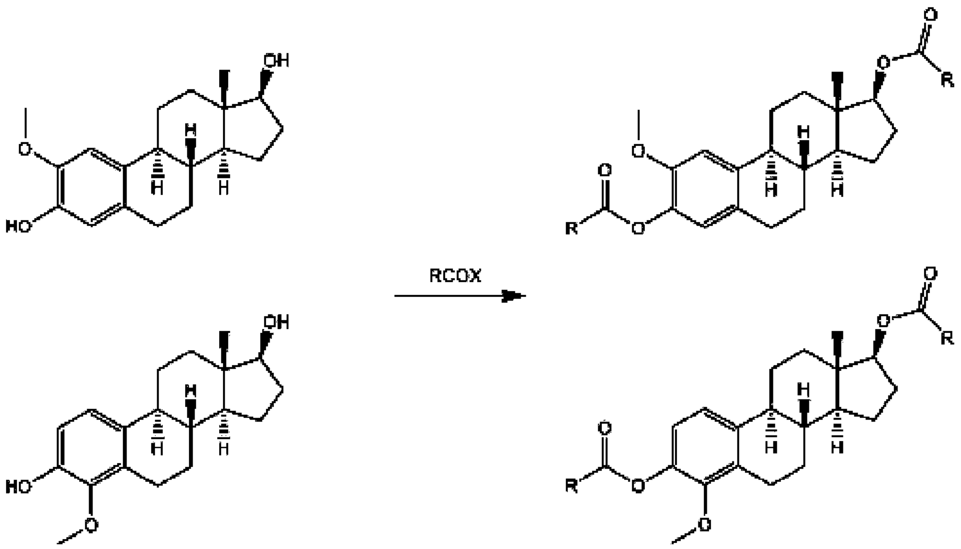


N-3 N-2"

wobei X im Fall von Estern O-Alkyl oder O-Aryl ist, im Fall von Anhydriden OCO-Alkyl ist und im Fall von Acylchloriden und Acylbromiden Cl oder Br ist;

b) Trennen des Derivats N-1' bzw. des Derivats N-1" aus dem Gemisch N-2' bzw. N-2" durch Kristallisation:

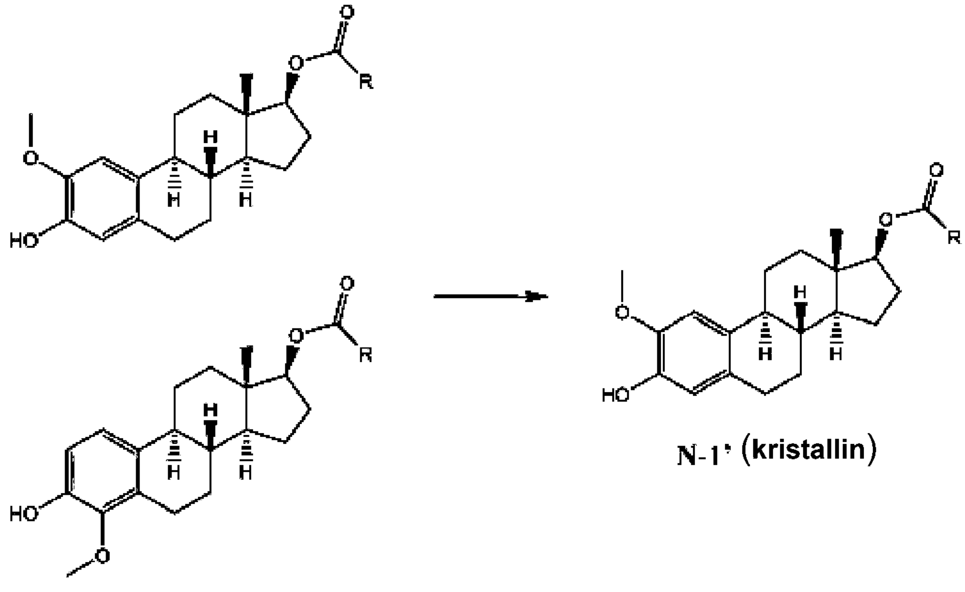

N-1' (kristallin)

N-2'

;

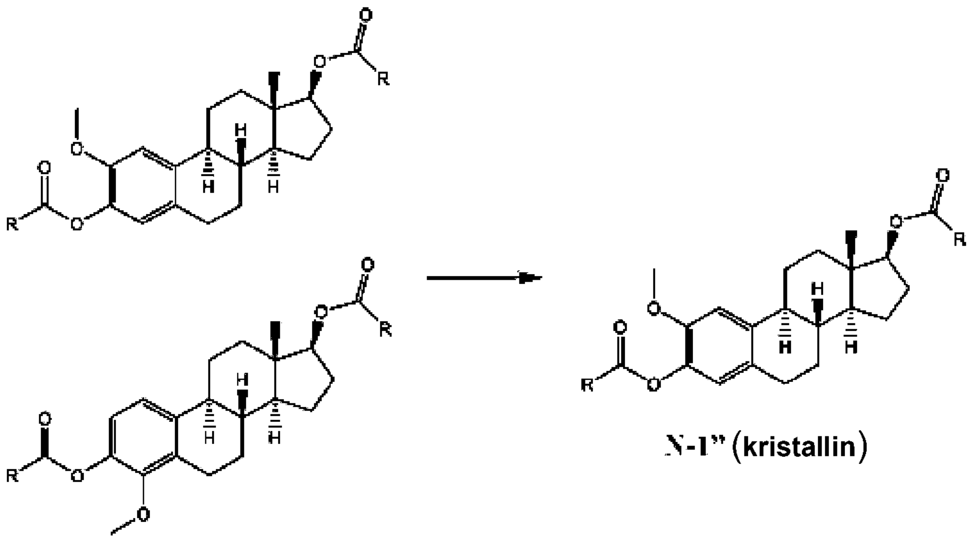

N-1" (kristallin)

N-2"

;

c) Hydrolysieren des Derivats N-1’ oder des Derivats N-1", um 2-Methoxyestradiol zu erhalten:

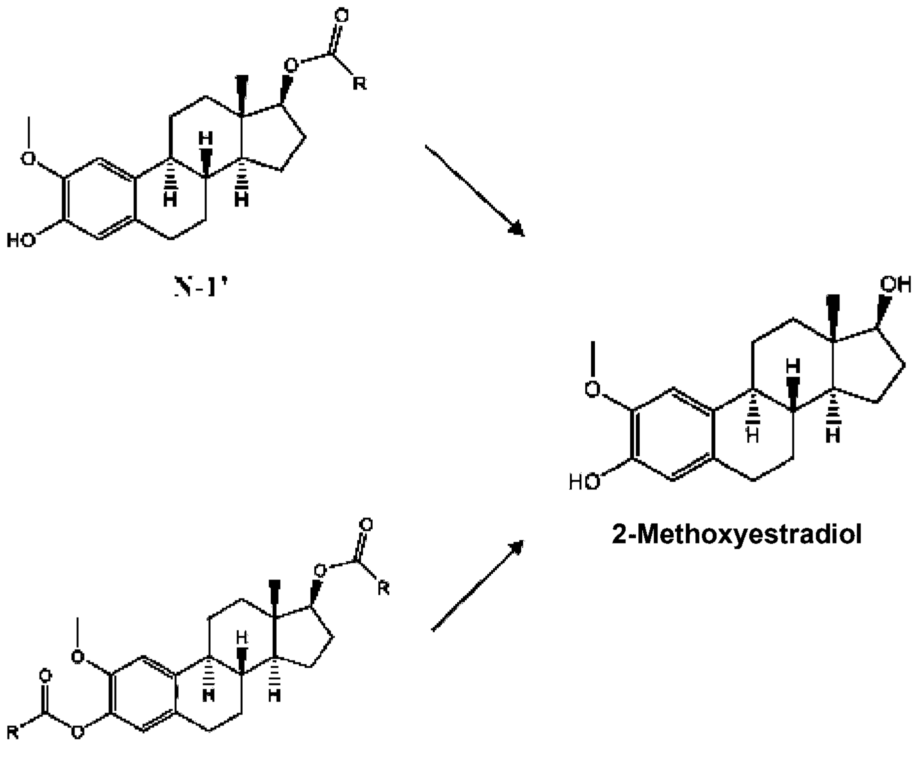

N-1'

2-Methoxyestradiol

N-1''

2. Verfahren nach Anspruch 1, wobei in Schritt a') die Säure aus Ameisensäure und Essigsäure ausgewählt ist.

3. Verfahren nach einem der Ansprüche 1 oder 2, wobei in Schritt a') die verwendete Säure als Lösungsmittel dient.

4. Verfahren nach einem der vorhergehenden Ansprüche, wobei in Schritt a') das Verhältnis von ml Säure zu Gramm N-3-Gemisch zwischen 4 und 8 liegt und der Schritt a') bei einer Temperatur zwischen 10 und 90 °C durchgeführt wird.

5. Verfahren nach einem der vorhergehenden Ansprüche, wobei in Schritt a') para-Toluolsulfonsäure als Katalysator verwendet wird.

6. Verfahren nach Anspruch 1, wobei Schritt a") mit einem Reagenzgemisch (N-3) durchgeführt wird, das 2-Methoxyestradiol und 4-Methoxyestradiol, ein organisches Lösungsmittel, eine organische Base, 4-Dimethylaminopyridin und das Carbonsäurederivat umfasst.

7. Verfahren nach Anspruch 6, wobei das Carbonsäurederivat aus Acylchloriden und -bromiden ausgewählt ist.

8. Verfahren nach Anspruch 7, wobei das Acylchlorid aus Benzoylchlorid und Furoylchlorid ausgewählt ist.

9. Verfahren nach einem der Ansprüche 6 bis 8, wobei Schritt a") bei einer Temperatur zwischen 10 und 50 °C durchgeführt wird, das Verhältnis zwischen dem Volumen des Lösungsmittels in ml und des Gewichts des N-3-Gemisches in g zwischen 10 und 25 liegt, das Verhältnis zwischen dem Volumen des Lösungsmittels und dem der organischen Base zwischen 3 und 6,5 liegt, das Volumen des Lösungsmittels mindestens das 10-Fache des Volumens des verwendeten Acylchlorids beträgt und das Verhältnis zwischen dem Gewicht des N-3-Gemisches und des Gewichts des 4-Dimethylaminopyridin-Katalysators zwischen 20 und 30 liegt.

10. Zwischenprodukt des Verfahrens nach Anspruch 1, ausgewählt aus:

- 2-Methoxy-3-hydroxyestra-1,3,5(10)-trien-17β-yl-formiat, das die folgende Formel aufweist:

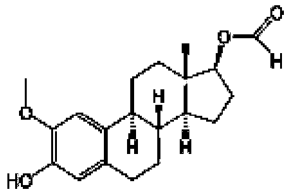

;

- 2-Methoxy-3-hydroxyestra-1,3,5(10)-trien-3,17β-yl-difuroat, das die folgende Formel aufweist:

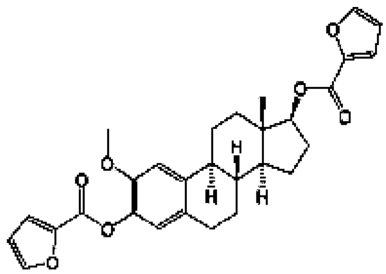

.

## Revendications

**1.** Procédé de purification du 2-méthoxyestradiol à partir du 4-méthoxyestradiol comprenant les étapes suivantes :

a) réaction d'un mélange (N-3) comprenant du 2-méthoxyestradiol et du 4-méthoxyestradiol avec :

a') un acide carboxylique choisi parmi l'acide formique, l'acide acétique et l'acide propionique ou
a") le dérivé d'un acide carboxylique choisi parmi un ester, un anhydride, un chlorure d'acyle et un bromure d'acyle,

obtenant :

- dans le cas a') un mélange N-2' comprenant les monoesters correspondants en position 17 du squelette stéroïdien :

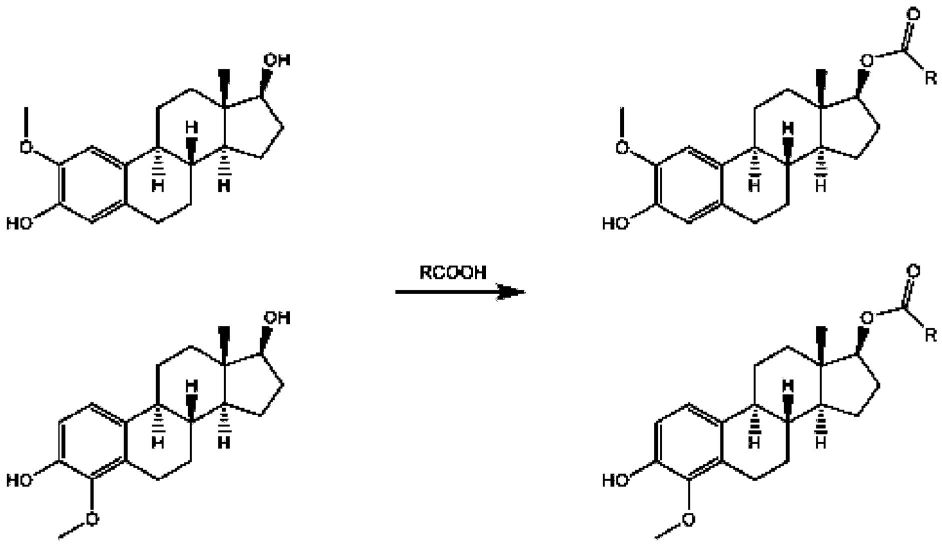


**N-3** **N-2'** ;

- dans le cas a") un mélange N-2" comprenant les diesters correspondants en positions 3 et 17 du squelette stéroïdien :

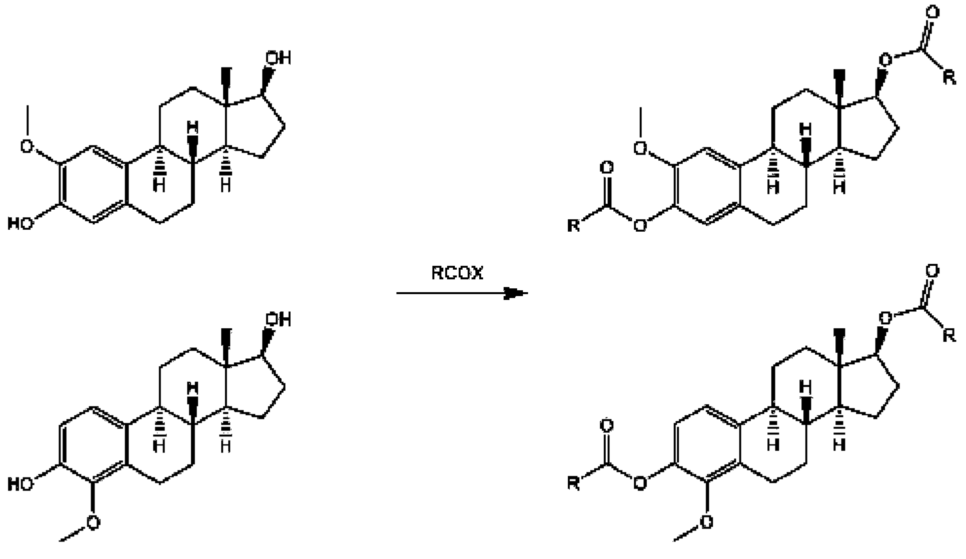


N-3 N-2"

où X est O-alkyle ou O-aryle dans le cas des esters, OCO-alkyle dans le cas des anhydrides, et Cl ou Br dans le cas des chlorures d'acyle et des bromures d'acyle ;

b) séparation par cristallisation du dérivé N-1' ou du dérivé N-1" du mélange N-2' ou N-2", respectivement :

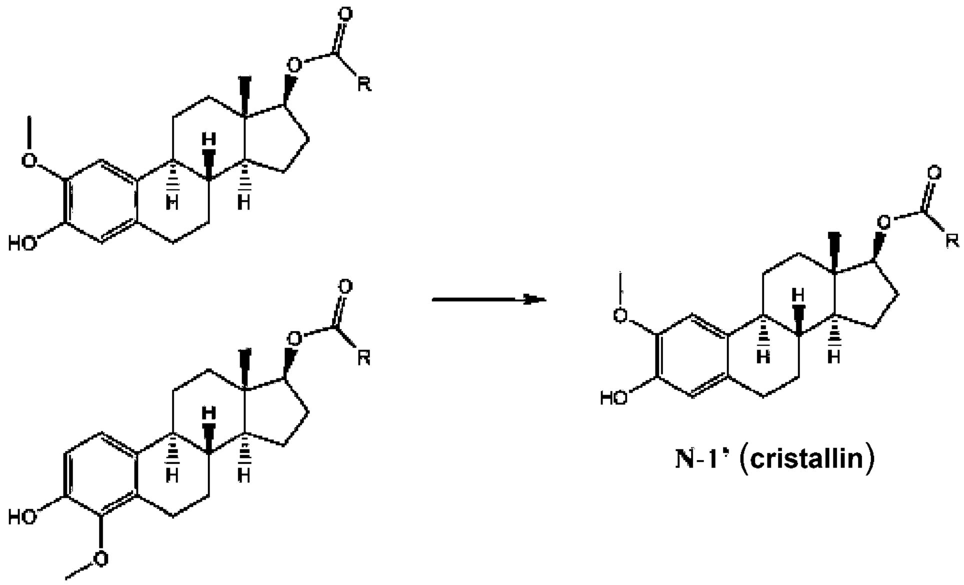

N-1' (cristallin)

N-2' ;

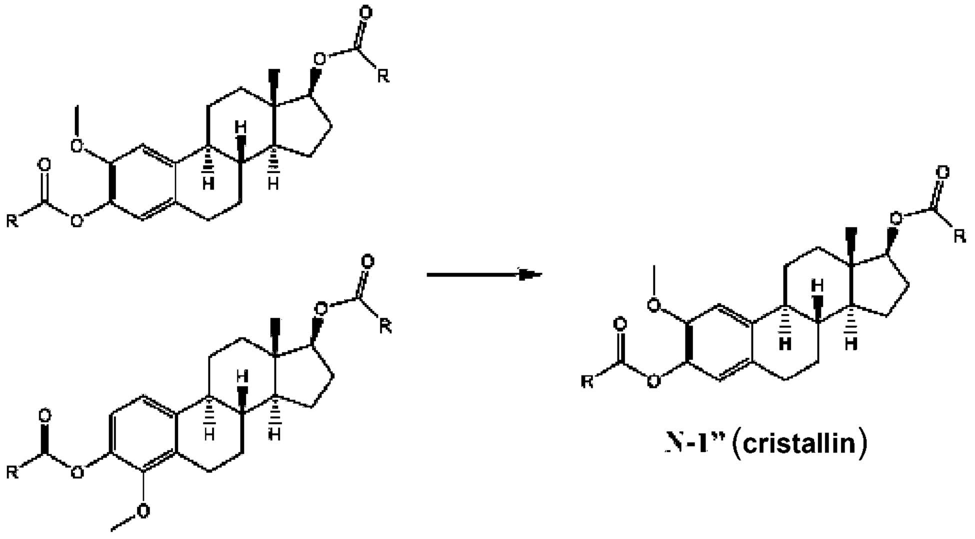

N-1'' (cristallin)

N-2''

;

c) hydrolyse du dérivé N-1’ ou du dérivé N-1" pour donner le 2-méthoxyestradiol:

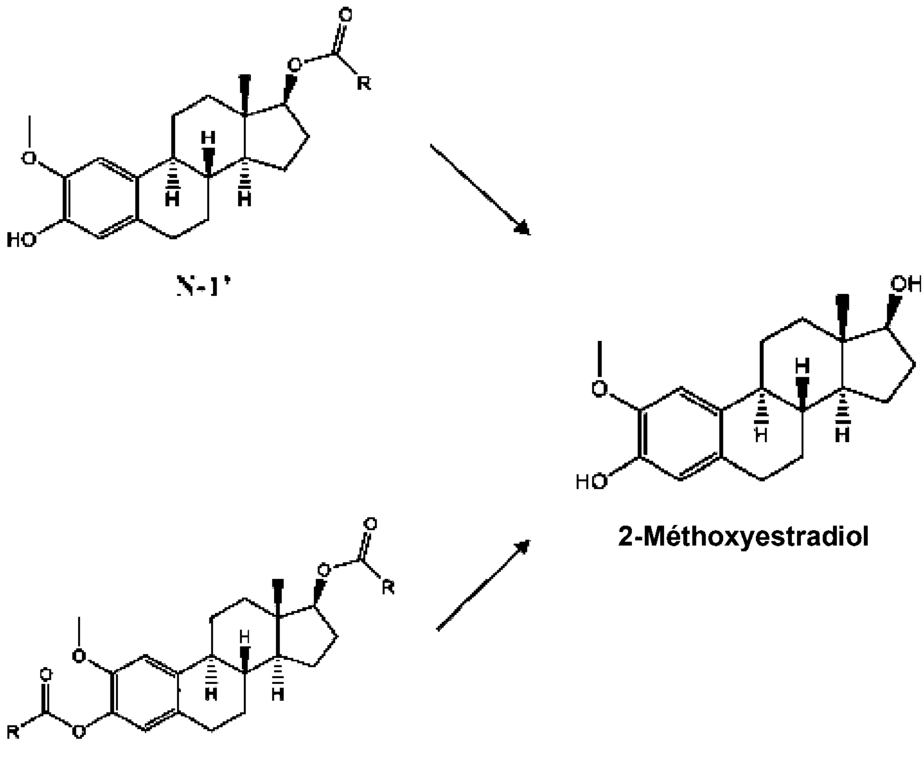

N-1'

2-Méthoxyestradiol

N-1''

.

2. Procédé selon la revendication 1, dans lequel dans l’étape a’) l’acide est choisi entre l’acide formique et l’acide acétique.

3. Procédé selon l’une quelconque des revendications 1 ou 2, dans lequel dans l’étape a’) l’acide utilisé agit comme un solvant.

4. Procédé selon l’une quelconque des revendications précédentes, dans lequel dans l’étape a’) le rapport entre ml d’acide et grammes du mélange N-3 est entre 4 et 8 et ladite étape a’) est réalisée à une température entre 10 et 90 °C.

5. Procédé selon l'une quelconque des revendications précédentes, dans lequel dans l'étape a') l'acide para-toluènesulfonique est utilisé comme catalyseur.

6. Procédé selon la revendication 1, dans lequel l'étape a") est réalisée avec un mélange de réactifs (N-3) comprenant le 2-méthoxyestradiol et le 4-méthoxyestradiol, un solvant organique, une base organique, la 4-diméthylaminopyridine et ledit dérivé d'un acide carboxylique.

7. Procédé selon la revendication 6, dans lequel ledit dérivé d'un acide carboxylique est choisi parmi les chlorures et les bromures d'acyle.

8. Procédé selon la revendication 7, dans lequel ledit chlorure d'acyle est choisi parmi le chlorure de benzoyle et le chlorure de furoyle.

9. Procédé selon l'une quelconque des revendications 6 à 8, dans lequel l'étape a") est réalisée à une température entre 10 et 50 °C, le rapport entre le volume du solvant en ml et le poids du mélange N-3 en g est entre 10 et 25, le rapport entre le volume du solvant et celui de la base organique est entre 3 et 6,5, le volume de solvant est au moins 10 fois le volume du chlorure d'acyle utilisé et le rapport entre le poids du mélange N-3 et le poids du catalyseur 4-diméthylaminopyridine est entre 20 et 30.

10. Intermédiaire du procédé selon la revendication 1, choisi parmi :

- formiate de 2-méthoxy-3-hydroxyestra-1,3,5 (10) -triène-17β-yle, ayant à la formule suivante :

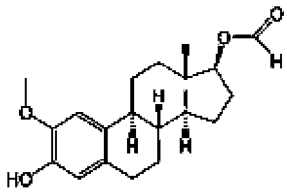

;

- difuroate de 2-méthoxy-3-hydroxyestra-1,3,5(10)-triène-3,17β-yle, ayant la formule suivante :

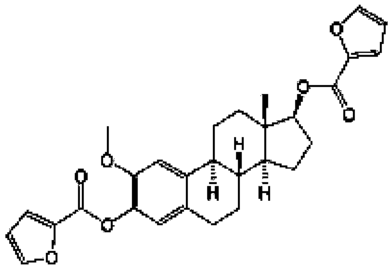

.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0114405 A2


### Non-patent literature cited in the description


- **V. A. FRANDSEN**. Demonstration of 2-methoxyestrone and of 2-methoxyestradiol in human pregnancy urine. *Acta Endocrinologica*, 1959, vol. 31, 603-7
- *CHEMICAL ABSTRACTS*, 362-07-2
- **T. H. LIPPERT et al.** The impact of endogenous estradiol metabolites on carcinogenesis. *Steroids*, 2000, vol. 65, 357-69
- **P. S. KIURU et al.** Short synthesis of 2-methoxyestradiol and 2-hydroxyestradiol. *Steroids*, 2003, vol. 68, 373-375
- *CHEMICAL ABSTRACTS*, 26788-23-8
- **Y. H. CHENG et al.** 4-Methoxyestradiol-induced oxidative injuries in human lung epithelial cells. *Toxicology and Applied Pharmacology*, 2007, vol. 220, 271-277
- **H.-H. LI et al.** Estradiol 17β and its metabolites stimulate cell proliferation and antagonize ascorbic acid-suppressed cell proliferation in human ovarian cancer cells. *Reproductive Sciences*, 2014, vol. 21, 102-111
- *Steroids*, 1986, vol. 47 (1), 63-81